# EUROPÄISCHE PATENTSCHRIFT

(11) **EP 1 613 745 B1**
(45) Veröffentlichungstag und Bekanntmachung des Hinweises auf die Patenterteilung: **19.03.2008**
(21) Anmeldenummer: 04725924.7
(22) Anmeldetag: 06.04.2004
(51) Int. Cl.: C12N 9/04

(54) **L-CARNITIN DEHYDROGENASEN, DEREN DERIVATE UND EIN VERFAHREN ZUR HERSTELLUNG VON SUBSTITUIERTEN (S)-ALKANOLEN**
L-CARNITIN DEHYDROGENASES, THEIR DERIVATIVES AND METHOD FOR PRODUCING SUBSTITUTED (S) ALKANOLS
L-CARNITINE DESHYDROGENASES, LEURS DERIVES ET PROCEDE DE PRODUCTION DE (S)-ALCANOLS SUBSTITUES

(30) Priorität: 07.04.2003 DE 10315760
(43) Veröffentlichungstag der Anmeldung: 11.01.2006
(73) Patentinhaber: BASF SE, 67056 Ludwigshafen (DE)
(72) Erfinder: ALTHÖFER, Henning, 67157 Wachenheim (DE); KESSELER, Maria, 68239 Mannheim (DE)
(74) Vertreter: Reitstötter - Kinzebach
(86) Internationale Anmeldenummer: PCT/EP2004/003655
(87) Internationale Veröffentlichungsnummer: WO 2004/090094

(56) Entgegenhaltungen:
- GB-A- 2 132 614
- LIU H ET AL: "CHEMO-ENZYMATIC SYNTHESIS OF THE ANTIDEPRESSANT DULOXETINE AND ITS ENANTIOMER" CHIRALITY, WILEY-LISS, NEW YORK, US, Bd. 12, Nr. 1, 2000, Seiten 26-29, XP009000316 ISSN: 0899-0042

### SEQUENCE LISTING

<110> BASF Aktiengesellschaft
<120> L-Carnitin Dehydrogenasen, deren Derivate und ein Verfahren zur Herstellung von substituierten (S)-Alkanolen
<130> M44098 CDH
<160> 10
<170> PatentIn version 3.1
<210> 1
   <211> 966
   <212> DNA
   <213> Alcaligenes sp.
<220>
   <221> CDS
   <222> (1)..(966)
   <223>
<400> 1
   atg act ttt atc acc aac atc aaa acc ttc gcc gct ctg ggc agc ggc        48
   Met Thr Phe Ile Thr Asn Ile Lys Thr Phe Ala Ala Leu Gly Ser Gly
   1               5                   10                  15
   gtg atc ggc agt ggc tgg gtg gcc cgc gcc ctg gcc cac ggc ctg gac        96
   Val Ile Gly Ser Gly Trp Val Ala Arg Ala Leu Ala His Gly Leu Asp
               20                  25                  30
   gtg gtg gcc tgg gac ccg gcg ccg ggc gct gaa gcc gcc ctg cgc cag       144
   Val Val Ala Trp Asp Pro Ala Pro Gly Ala Glu Ala Ala Leu Arg Gln
            35                  40                  45
   cgg gtc gcc aac gcc tgg ggc gct ctg gaa aaa cag ggc ctg gtc ccc      192
   Arg Val Ala Asn Ala Trp Gly Ala Leu Glu Lys Gln Gly Leu Val Pro
        50                  55                  60
   gga gcc tcg cca cag cgc cta cgc ttt gtc agc acc atc gaa gag tgt      240
   Gly Ala Ser Pro Gln Arg Leu Arg Phe Val Ser Thr Ile Glu Glu Cys
   65                  70                  75                  80
   gtg aaa gac gcc gac ttc atc cag gaa agc gct ccc gag cgc ctg gaa      288
   Val Lys Asp Ala Asp Phe Ile Gln Glu Ser Ala Pro Glu Arg Leu Glu
                    85                  90                  95
   ctc aag ctg gaa ctg cac agc aag atc agc gcc gcg gcc aag ccc gac      336
   Leu Lys Leu Glu Leu His Ser Lys Ile Ser Ala Ala Ala Lys Pro Asp
               100                 105                 110
   gcc ctg atc ggc tcc agc acc tcg gcc ctg ctg ccc agc gaa ttc tat      384
   Ala Leu Ile Gly Ser Ser Thr Ser Ala Leu Leu Pro Ser Glu Phe Tyr
           115                 120                 125
   gag ggc tcg acc cac ccg caa cgc tgc gtg gtg ggc cat ccg ttc aac      432
   Glu Gly Ser Thr His Pro Gln Arg Cys Val Val Gly His Pro Phe Asn
       130                 135                 140
   ccg gtg tac ctg cta ccg ctg gtg gaa gtg gtg ggc ggc cag cac acc      480
   Pro Val Tyr Leu Leu Pro Leu Val Glu Val Val Gly Gly Gln His Thr
   145                 150                 155                 160
   gca ccc gag gcg ata cag gcg gcg atc cag gtc tat gaa tcc ttg ggg      528
   Ala Pro Glu Ala Ile Gln Ala Ala Ile Gln Val Tyr Glu Ser Leu Gly
                   165                 170                 175
   atg cgc ccc ttg cat gtg cgc aag gaa gtc ccg ggc ttc atc gcc gac      576
   Met Arg Pro Leu His Val Arg Lys Glu Val Pro Gly Phe Ile Ala Asp
               180                 185                 190
   cgt ctg ctc gaa gcc ctg tgg cgc gaa gcc ttg cac ctg gtc aat gac      624
   Arg Leu Leu Glu Ala Leu Trp Arg Glu Ala Leu His Leu Val Asn Asp
           195                 200                 205
   ggg gtg gcg acc acc ggg gaa atc gac gac gcg atc cgc ttt ggc gcc      672
   Gly Val Ala Thr Thr Gly Glu Ile Asp Asp Ala Ile Arg Phe Gly Ala
           210                     215                     220
   ggc ctg cgc tgg tcg ttc atg ggc acc ttc ctc acc tac acc ctg gcc      720
   Gly Leu Arg Trp Ser Phe Met Gly Thr Phe Leu Thr Tyr Thr Leu Ala
   225                 230                 235                 240
   gga ggc gac gcc ggc atg cgt cac ttc atg gcc cag ttc ggc ccg gcg      768
   Gly Gly Asp Ala Gly Met Arg His Phe Met Ala Gln Phe Gly Pro Ala
                   245                 250                 255
   ctg caa ctg ccc tgg acc tac ctg ccg gcc ccg gag ctg acc gac aaa      816
   Leu Gln Leu Pro Trp Thr Tyr Leu Pro Ala Pro Glu Leu Thr Asp Lys
               260                 265                 270
   ctg atc gat gac gta gtc caa ggt acc gcc gag caa ctg ggc aac cac      864
   Leu Ile Asp Asp Val Val Gln Gly Thr Ala Glu Gln Leu Gly Asn His
           275                 280                 285
   agc att tcg gcc ctg gag cgc tat cgt gat gac tgc ctg ctg gcg gtg      912
   Ser Ile Ser Ala Leu Glu Arg Tyr Arg Asp Asp Cys Leu Leu Ala Val
       290                 295                 300
   ctg gag gcg gtg aaa acc acc aag gcc aag cac ggc atg cac ttt cac      960
   Leu Glu Ala Val Lys Thr Thr Lys Ala Lys His Gly Met His Phe His
   305                 310                 315                 320
   gac taa                                                              966
   Asp
<210> 2
   <211> 321
   <212> PRT
   <213> Alcaligenes sp.
<400> 2
   Met Thr Phe Ile Thr Asn Ile Lys Thr Phe Ala Ala Leu Gly Ser Gly
   1               5                   10                  15
   Val Ile Gly Ser Gly Trp Val Ala Arg Ala Leu Ala His Gly Leu Asp
               20                  25                  30
   Val Val Ala Trp Asp Pro Ala Pro Gly Ala Glu Ala Ala Leu Arg Gln
           35                  40                  45
   Arg Val Ala Asn Ala Trp Gly Ala Leu Glu Lys Gln Gly Leu Val Pro
       50                  55                  60
   Gly Ala Ser Pro Gln Arg Leu Arg Phe Val Ser Thr Ile Glu Glu Cys
   65                  70                  75                  80
   Val Lys Asp Ala Asp Phe Ile Gln Glu Ser Ala Pro Glu Arg Leu Glu
                   85                  90                  95
   Leu Lys Leu Glu Leu His Ser Lys Ile Ser Ala Ala Ala Lys Pro Asp
               100                 105                 110
   Ala Leu Ile Gly Ser Ser Thr Ser Ala Leu Leu Pro Ser Glu Phe Tyr
           115                 120                 125
   Glu Gly Ser Thr His Pro Gln Arg Cys Val Val Gly His Pro Phe Asn
       130                 135                 140
   Pro Val Tyr Leu Leu Pro Leu Val Glu Val Val Gly Gly Gln His Thr
   145                 150                 155                 160
   Ala Pro Glu Ala Ile Gln Ala Ala Ile Gln Val Tyr Glu Ser Leu Gly
                   165                 170                 175
   Met Arg Pro Leu His Val Arg Lys Glu Val Pro Gly Phe Ile Ala Asp
               180                 185                 190
   Arg Leu Leu Glu Ala Leu Trp Arg Glu Ala Leu His Leu Val Asn Asp
           195                 200                 205
   Gly Val Ala Thr Thr Gly Glu Ile Asp Asp Ala Ile Arg Phe Gly Ala
       210                 215                 220
   Gly Leu Arg Trp Ser Phe Met Gly Thr Phe Leu Thr Tyr Thr Leu Ala
   225                 230                 235                 240
   Gly Gly Asp Ala Gly Met Arg His Phe Met Ala Gln Phe Gly Pro Ala
                   245                 250                 255
   Leu Gln Leu Pro Trp Thr Tyr Leu Pro Ala Pro Glu Leu Thr Asp Lys
               260                 265                 270
   Leu Ile Asp Asp Val Val Gln Gly Thr Ala Glu Gln Leu Gly Asn His
           275                 280                 285
   Ser Ile Ser Ala Leu Glu Arg Tyr Arg Asp Asp Cys Leu Leu Ala Val
       290                 295                 300
   Leu Glu Ala Val Lys Thr Thr Lys Ala Lys His Gly Met His Phe His
   305                 310                 315                 320
   Asp
<210> 3
   <211> 321
   <212> PRT
   <213> Pseudomonas putida
<400> 3
   Met Pro Phe Ile Thr Glu Ile Lys Thr Phe Ala Ala Leu Gly Ser Gly
   1 5 10 15
   Val Ile Gly Ser Gly Trp Val Ala Arg Ala Leu Ala His Gly Leu Asp
   20 25 30
   Val Val Ala Trp Asp Pro Ala Pro Gly Ala Glu Gln Ala Leu Arg Lys
   35 40 45
   Arg Val Ala Asn Ala Trp Pro Ala Leu Glu Lys Gln Gly Leu Ala Pro
   50 55 60
   Gly Ala Ser Gln Asp Arg Leu Lys Phe Val Ala Thr Ile Glu Glu Cys
   65 70 75 80
   Val Arg Asn Ala Asp Phe Ile Gln Glu Ser Ala Pro Glu Arg Leu Asp
   85 90 95
   Leu Lys Leu Asp Leu His Ala Lys Ile Ser Ala Ala Ala Lys Pro Asp
   100 105 110
   Ala Ile Ile Gly Ser Ser Thr Ser Gly Leu Leu Pro Ser Glu Phe Tyr
   115 120 125
   Glu Ser Ser Thr His Pro Glu Arg Cys Val Val Gly His Pro Phe Asn
   130 135 140
   Pro Val Tyr Leu Leu Pro Leu Val Glu Ile Val Gly Gly Ser Arg Thr
   145 150 155 160
   Ser Pro Glu Ala Ile Glu Ala Ala Lys Thr Ile Tyr Thr Ala Leu Gly
   165 170 175
   Met Arg Pro Leu His Val Arg Lys Glu Val Pro Gly Phe Ile Ala Asp
   180 185 190
   Arg Leu Leu Glu Ala Leu Trp Arg Glu Ala Leu His Leu Val Asn Asp
   195 200 205
   Gly Val Ala Thr Thr Gly Glu Ile Asp Asp Ala Ile Arg Phe Gly Ala
   210 215 220
   Gly Leu Arg Trp Ser Phe Met Gly Thr Phe Leu Thr Tyr Thr Leu Ala
   225 230 235 240
   Gly Gly Asp Ala Gly Met Arg His Phe Met Ser Gln Phe Gly Pro Ala
   245 250 255
   Leu Lys Leu Pro Trp Thr Tyr Leu Pro Ala Pro Glu Leu Thr Asp Lys
   260 265 270
   Leu Ile Asp Asp Val Val Ser Gly Thr Ser Glu Gln Gln Gly Glu Arg
   275 280 285
   Ser Ile Ala Ala Leu Glu Arg Tyr Arg Asp Asp Thr Leu Leu Ala Val
   290 295 300
   Leu Glu Ala Val Lys Ser Ser Lys Ala Ser His Gly Leu Ser Phe Ser
   305 310 315 320
   Asp
<210> 4
   <211> 321
   <212> PRT
   <213> Pseudomonas aeruginosa
<400> 4
   Met Ser Phe Val Thr Glu Ile Lys Thr Phe Ala Ala Leu Gly Ser Gly
   1               5                   10                  15
   Val Ile Gly Ser Gly Trp Ile Ala Arg Ala Leu Ala His Gly Leu Asp
               20                  25                  30
   Val Val Ala Trp Asp Pro Ala Pro Gly Ala Glu Ala Ala Leu Arg Ala
           35                  40                  45
   Arg Val Ala Asn Ala Trp Pro Ala Leu Arg Lys Gln Gly Leu Ala Pro
       50                  55                  60
   Gly Ala Ala Gln Glu Arg Leu Arg Phe Val Ala Ser Ile Glu Glu Cys
   65                  70                  75                  80
   Val Gly Asp Ala Asp Phe Ile Gln Glu Ser Ala Pro Glu Arg Leu Asp
                   85                  90                  95
   Leu Lys Leu Asp Leu His Ala Arg Ile Ser Ala Ala Ala Arg Pro Asp
               100                 105                 110
   Val Leu Ile Gly Ser Ser Thr Ser Gly Leu Leu Pro Ser Glu Phe Tyr
           115                 120                 125
   Ala Glu Ala Ser His Pro Glu Arg Cys Leu Val Gly His Pro Phe Asn
       130                 135                 140
   Pro Val Tyr Leu Leu Pro Leu Val Glu Val Val Gly Gly Glu Arg Thr
   145 150 155 160
   Ala Ala Glu Ala Val Arg Ala Ala Met Arg Val Tyr Glu Ser Leu Gly
   165 170 175
   Met Arg Pro Leu His Val Arg Lys Glu Val Pro Gly Phe Ile Ala Asp
   180 185 190
   Arg Leu Leu Glu Ala Leu Trp Arg Glu Ala Leu His Leu Val Asn Asp
   195 200 205
   Gly Val Ala Thr Thr Gly Glu Ile Asp Asp Ala Ile Arg Phe Gly Ala
   210 215 220
   Gly Leu Arg Trp Ser Phe Met Gly Thr Phe Leu Thr Tyr Thr Leu Ala
   225 230 235 240
   Gly Gly Asn Ala Gly Met Arg His Phe Met Ala Gln Phe Gly Pro Ala
   245 250 255
   Leu Gln Leu Pro Trp Thr Tyr Leu Pro Ala Pro Glu Leu Thr Glu Ala
   260 265 270
   Leu Ile Asp Arg Val Val Glu Gly Thr Ala Glu Gln Gln Gly Ala Arg
   275 280 285
   Ser Ile Ala Glu Leu Glu Arg Tyr Arg Asp Asp Cys Leu Leu Ala Val
   290 295 300
   Leu Gly Ala Ile Arg Glu Thr Lys Ala Arg His Gly Phe Ala Phe Ala
   305 310 315 320
   Glu
<210> 5
   <211> 256
   <212> PRT
   <213> Xanthomonas campestris
<400> 5
   Met Gln Pro Ser Ser Val Leu Ala Ile Val Thr Gly Gly Val Ser Gly
   1               5                   10                  15
   Leu Gly Leu Ala Val Ala Gln Arg Ile Val Ala Asp Gly Gly Arg Val
               20                  25                  30
   Ala Leu Phe Asp Val Asn Ala Asp Lys Ala Asp Ala Ala Leu Ala Leu
           35                  40                  45
   Leu Gly Ala Gly Asn Ala His Tyr Phe Ala Thr Asp Val Thr Asp Glu
       50                  55                  60
   Ala Gln Val Ala Ala Asn Val Ala Gln Ala Ala Gln Ala Leu Gly Gly
   65                  70                  75                  80
   Leu Asn Leu Ala Val Asn Cys Ala Gly Ile Leu Gly Ala Gly Arg Val
                   85                  90                  95
   Leu Gly Lys Glu Ala Ala Met Pro Leu Ala Thr Phe Arg Thr Thr Val
               100                 105                 110
   Met Val Asn Leu Val Gly Ser Phe Asn Val Ala Lys Ala Ala Ala Asp
   115 120 125
   Val Met Gln His Asn Ala Ala Gly Glu Asp Gly Glu Arg Gly Val Ile
   130 135 140
   Ile His Thr Ala Ser Val Ala Ala Tyr Glu Gly Gln Ile Gly Gln Ala
   145 150 155 160
   Ala Tyr Ala Ala Ser Lys Gly Gly Val Val Gly Met Thr Leu Pro Met
   165 170 175
   Ala Arg Glu Leu Ala Arg Phe Gly Ile Arg Val Met Thr Ile Ala Pro
   180 185 190
   Gly Val Phe Trp Thr Pro Met Leu Asp Gly Met Pro Glu Thr Val Gln
   195 200 205
   Gln Ser Leu Ala Ala Ser Ile Pro Phe Pro Ser Arg Leu Gly Gln Pro
   210 215 220
   Asp Glu Tyr Ala Glu Thr Val Ala Phe Ile Leu Arg Thr Arg Tyr Leu
   225 230 235 240
   Asn Gly Glu Val Ile Arg Leu Asp Gly Gly Val Arg Leu Ala Pro Lys
   245 250 255
<210> 6
   <211> 321
   <212> PRT
   <213> Staphylococcus epidermidis
<400> 6
   Met Lys Phe Ala Val Val Gly Thr Gly Val Ile Gly Ser Gly Trp Ile
   1 5 10 15
   Thr Arg Met Leu Ala His Gly His Glu Val Ile Ala Thr Asp Pro Ser
   20 25 30
   Glu Gly Ala Tyr Glu Arg Met Leu Thr Gln Val Lys Gln Asn Trp Pro
   35 40 45
   Tyr Ala Glu Gln Met Gly Leu Ala Glu Asn Ala Ser Ile Gln Asn Leu
   50 55 60
   Thr Phe Thr Pro His Leu Glu Glu Ala Val Lys Asp Ala Asp His Ile
   65 70 75 80
   Gln Glu Asn Val Pro Glu Val Glu Glu Ile Lys Asp Ala Val Leu Lys
   85 90 95
   Glu Ile Asp Phe Tyr Ala Lys Pro Glu Ala Thr Ile Gly Ser Ser Thr
   100 105 110
   Ser Gly Ile Met Pro Ser Glu Leu Gln Ala Asn Leu Ser His Pro Glu
   115 120 125
   Arg Leu Val Val Ala His Pro Phe His Pro Val Tyr Ile Leu Pro Leu
   130 135 140
   Val Glu Ile Val Pro Gly Lys Gln Thr Ser Glu Glu Thr Thr Val Lys
   145 150 155 160
   Ala Glu Gln Ile Tyr Glu Ser Ile Gly Met Asp Val Leu His Val Arg
   165 170 175
   His Glu Ile Glu Gly His Ile Ala Asp Arg Leu Met Glu Ala Leu Trp
   180 185 190
   Arg Glu Ser Leu His Ile Val Asn Asp Gly Ile Ala Thr Thr Glu Glu
   195 200 205
   Val Asp Lys Ala Phe Thr His Ala Ala Gly Leu Arg Tyr Ala Gln Tyr
   210 215 220
   Gly Pro Phe Met Thr Phe His Leu Ala Gly Gly Glu Gly Gly Met Arg
   225 230 235 240
   His Met Leu Lys Gln Phe Gly Pro Ala Leu Lys Lys Pro Trp Thr Lys
   245 250 255
   Leu Ile Ala Pro Glu Leu Thr Asp Asp Leu Tyr His Lys Val Val Ser
   260 265 270
   Gly Ser Glu Ala Ser Ser Gln Gly Tyr Thr Met Ser Glu Leu Asp Gln
   275 280 285
   Lys Arg Asn Glu Phe Leu Ile Lys Val Lys Glu Leu Ala Glu Gln Tyr
   290 295 300
   Trp Pro Ser Asp Ser Lys Ala Met Lys Lys Ser Asn Gly Ala Glu Leu
   305 310 315 320
   Gln
<210> 7
   <211> 364
   <212> PRT
   <213> Rhizobium loti
<400> 7
   Met Ser Ile Ile Asn Lys Ala Ala Ala Ile Gly Gly Gly Val Ile Gly
   1               5                   10                  15
   Ala Gly Trp Val Ala Arg Leu Leu Leu Asn Gly Ile Asp Val Ser Ile
               20                  25                  30
   Phe Asp Pro Asp Pro Glu Ala Ser Arg Lys Val Ser Glu Val Met Lys
           35                  40                  45
   Gly Ala Arg Arg Ala Tyr Lys Gln Met Val Pro Gly Gly Leu Pro Lys
       50                  55                  60
   Glu Gly Lys Leu Thr Phe Ala Lys Thr Ile Ala Glu Ala Val Ala Asp
   65                  70                  75                  80
   Ala Asp Phe Ile Gln Glu Ser Val Pro Glu Arg Leu Asp Leu Lys His
                   85                  90                  95
   Arg Val Leu Ala Glu Ile Asp Ala His Ala Pro Ala Asn Ala Ile Val
               100                 105                 110
   Gly Ser Ser Thr Ser Gly Ile Lys Pro Thr Asp Met Gln Val Ala Met
           115                 120                 125
   Lys Lys His Pro Glu Arg Leu Val Val Gly His Pro Phe Asn Pro Val
       130                 135                 140
   Tyr Leu Leu Pro Leu Val Glu Ile Val Gly Gly Asp Gln Thr Phe Pro
   145 150 155 160
   Glu Ala Ile Glu Val Ala Lys Glu Ile Tyr Ala Ser Ile Gly Met Lys
   165 170 175
   Pro Val Val Ile Arg Lys Glu Ile Glu Ala Phe Val Gly Asp Arg Leu
   180 185 190
   Leu Glu Ala Ala Trp Arg Glu Ala Leu Trp Leu Ile Lys Asp Gly Ile
   195 200 205
   Cys Thr Val Glu Glu Leu Asp Asp Ile Met Arg Tyr Gly Phe Gly Leu
   210 215 220
   Arg Trp Ala Gln Met Gly Met Phe Gln Val Tyr Arg Val Ala Gly Gly
   225 230 235 240
   Glu Ala Gly Met Arg His Phe Met Ala Gln Phe Gly Pro Cys Leu Lys
   245 250 255
   Trp Pro Trp Thr Lys Leu Met Asp Val Pro Glu Phe Asn Asp Glu Leu
   260 265 270
   Val Asp Leu Ile Ala Thr Gln Ser Asp Asp Gln Ala His Gly Leu Ser
   275 280 285
   Ile Arg Glu Leu Glu Lys Ile Arg Asp Asp Asn Leu Val Ala Ile Met
   290 295 300
   Asp Ala Leu Ser Lys Gln Asn Lys Gly Lys Gly Trp Gly Ala Gly Ala
   305 310 315 320
   Leu His Lys Asp Tyr Thr Lys Gln Leu Ala Lys Leu Ala Ala Lys Lys
   325 330 335
   Pro Ala Ala Ser Thr Ala Ala Glu Lys Ala Lys Ala Ser Lys Pro Val
               340                 345                 350
   Lys Lys Ala Glu Lys Pro Lys Lys Lys Lys Lys Gly
           355                 360
<210> 8
   <211> 488
   <212> PRT
   <213> Agrobacterium tumefaciens
<400> 8
   Met Val Ala Gly Met Lys Ile Ala Ala Ile Gly Gly Gly Val Ile Gly
   1               5                   10                  15
   Gly Gly Trp Ile Ala Arg Phe Ile Leu Ala Gly His Asp Val Thr Val
               20                  25                  30
   Phe Asp Pro His Pro Glu Ala Asn Arg Ile Val Thr Glu Val Met Ala
           35                  40                  45
   Asn Ala Ala Glu Ala Trp Gly Arg Leu Tyr Gln Ser Pro Leu Pro Lys
       50                  55                  60
   Pro Gly Ser Ile Asn Trp Ala Ser Ser Ile Ala Glu Ala Val Ala Gly
   65                  70                  75                  80
   Ala Asp Tyr Ile Gln Glu Ser Val Pro Glu Arg Leu Asp Leu Lys His
                   85                  90                  95
   Arg Ile Ile Ala Glu Ile Glu Ala Thr Ala Ser Pro Gln Ala Ile Ile
   100 105 110
   Ala Ser Ser Thr Ser Gly Phe Lys Pro Ser Glu Leu Arg Glu Gly Ser
   115 120 125
   Val His Ser Glu Arg Val Ile Val Ala His Pro Phe Asn Pro Val Tyr
   130 135 140
   Leu Leu Pro Val Val Glu Val Val Gly Gly Gly Val Ala Ala Gln Arg
   145 150 155 160
   Ala Ser Asp Ile Leu Val Ser Val Gly Met Lys Pro Val Gln Ile Gly
   165 170 175
   Arg Glu Ile Asp Ala His Ile Gly Asp Arg Leu Leu Glu Ala Ile Trp
   180 185 190
   Arg Glu Ala Leu Trp Leu Val Lys Asp Gly Ile Ala Thr Thr Gln Glu
   195 200 205
   Ile Asp Asp Ile Ile Arg Tyr Gly Phe Gly Leu Arg Trp Ala Gln Met
   210 215 220
   Gly Leu Phe Glu Thr Tyr Arg Ile Ala Gly Gly Glu Ala Gly Met Arg
   225 230 235 240
   His Phe Leu Ala Gln Phe Gly Pro Ala Leu Lys Trp Pro Trp Thr Lys
   245 250 255
   Leu Met Asp Val Pro Glu Phe Asn Asp Glu Leu Ile Asp Leu Ile Ala
   260 265 270
   Gly Gln Ser Asp Ala Gln Ser Gly Ala Tyr Ser Ile Arg Glu Leu Glu
           275                 280                 285
   Arg Ile Arg Asp Ser Asn Leu Ile Gly Ile Phe His Ala Leu Lys Ala
       290                 295                 300
   Asn Asp Trp Gly Ala Gly Gln Thr Val Lys Ala Met Glu Asn Arg Phe
   305                 310                 315                 320
   Tyr Ala Arg Asn Gly Lys Val Gln Ala Asp Tyr Pro Leu Arg Leu His
                   325                 330                 335
   Glu Ala His Val Asn Gly Gly Trp Val Asp Tyr Asn Gly His Met Thr
               340                 345                 350
   Glu Phe Arg Tyr Leu Gln Val Leu Gly Asp Ala Thr Asp Ala Leu Leu
           355                 360                 365
   Ile His Ile Gly Leu Asp Ala Asp Tyr Arg Ala Ala Gly His Ser Ala
       370                 375                 380
   Tyr Thr Val Glu Thr His Ile Arg His Leu Ala Glu Val Lys Ala Gly
   385                 390                 395                 400
   Ala Arg Leu Thr Val Glu Thr Arg Leu Leu Gly Tyr Asp Asp Lys Arg
                   405                 410                 415
   Leu Arg Leu His His Ala Ile Leu Asn Glu Asp Gly Glu Thr Val Ala
               420                 425                 430
   Thr Gly Glu His Met Leu Leu His Val Asp Thr Lys Ala Asn Arg Thr
           435                 440                 445
   Val Ala Met Pro Pro Ala Leu Met Arg Ala Leu Asp His Leu Asn Ala
       450                 455                 460
   Gln Glu Glu Gly Pro Leu Pro Asp His Ala Gly Ser Gly Ile Arg Ala
   465                 470                 475                 480
   Val Arg Leu Lys Glu Thr Ser Ala
                   485
<210> 9
   <211> 307
   <212> PRT
   <213> Streptomyces coelicolor
<400> 9
   Met Ser Thr Val Thr Val Ile Gly Ala Gly Thr Ile Gly Leu Gly Trp
   1 5 10 15
   Ile Asn Leu Phe Ser Ala Arg Gly Leu Thr Val Arg Val Asn Ser Arg
   20 25 30
   Arg Pro Asp Val Arg Arg Val Val His Glu Ala Leu Glu Leu Phe Ser
   35 40 45
   Pro Gly Arg Val Asp Glu Leu Ala Ala Arg Ile Glu Tyr Glu Pro Asp
   50 55 60
   Val Gly Arg Ala Val Ala Gly Ala Asp Val Val Ser Glu Asn Ala Pro
   65 70 75 80
   Asp Asp Leu Pro Leu Lys Gln Arg Leu Phe Ala Glu Ile Gly Ala Ala
   85 90 95
   Ala Pro Asp His Ala Leu Val Leu Ser Ser Thr Ser Lys Leu Leu Pro
   100 105 110
   Asp Glu Leu Ser Arg Asp Met Pro Gly Pro Gly Arg Leu Val Val Ala
   115 120 125
   His Pro Phe Asn Pro Pro His Ile Val Pro Leu Val Glu Val Val Arg
   130 135 140
   Gly Glu Arg Thr Asp Pro Glu Ala Val Glu Arg Thr Leu Ala Phe Leu
   145 150 155 160
   Ala Ser Val Gly Arg Thr Pro Val Val Val Arg Arg Ala Leu Pro Gly
   165 170 175
   Phe Ala Ala Asn Arg Leu Gln Ser Ala Leu Leu Arg Glu Ser Ile His
   180 185 190
   Leu Val Leu Glu Gly Val Val Thr Val Glu Glu Leu Asp Arg Ile Val
   195 200 205
   Thr Asp Ser Ile Gly Leu Arg Trp Ser Thr Ile Gly Pro Phe His Ala
   210 215 220
   Phe His Leu Gly Gly Gly Pro Gly Gly Leu Arg Lys Trp Leu Glu His
   225 230 235 240
   Leu Gly Ser Gly Leu Glu Gln Gly Trp Arg Gly Leu Gly Gln Pro Ala
   245 250 255
   Leu Thr Pro Gln Ala Val Glu Ala Leu Val Ala Gln Thr Glu Ala Ala
   260 265 270
   Tyr Gly His Arg Pro Tyr Ala Glu Leu Val Arg Asp Arg Asp Asp Arg
   275 280 285
   His Leu Ala Val Leu Ala Ala Leu Glu Arg Thr Glu Gln Pro Gln Glu
   290 295 300
   Glu Thr Lys
   305
<210> 10
   <211> 315
   <212> PRT
   <213> Archaeoglobus fulgidus
<400> 10
   Met Gly Val Lys Val Ala Cys Ile Gly Ala Gly Thr Val Gly Ala Ser
   1 5 10 15
   Trp Ala Ser Leu Phe Ala Trp Arg Gly Cys Asp Val Ala Val Tyr Asp
   20 25 30
   Pro Phe Pro Glu Ala Leu Asn Arg Ala Glu Ala Ser Ile Ala Arg Thr
   35 40 45
   Val Ser Thr Leu Ser Glu Ile Phe Ser Gly Ser Glu Asp Asp Val Lys
   50 55 60
   Ser Ala Leu Ser Arg Val Lys Phe Thr Glu Asn Leu Glu Glu Ala Leu
   65 70 75 80
   Lys Gly Ala Tyr Tyr Val Gln Glu Ser Ala Val Glu Lys Leu Glu Val
   85 90 95
   Lys Arg Asp Leu Phe Glu Lys Met Asp Ala Ile Ala Glu Pro Glu Thr
   100 105 110
   Ile Leu Ala Thr Ser Thr Ser Gly Leu Ser Ile Ser Glu Ile Gln Thr
   115 120 125
   Ala Ala Arg Lys His Pro Glu Arg Cys Ile Thr Ala His Pro Tyr Asn
   130 135 140
   Pro Pro His Leu Ile Pro Leu Val Glu Val Val Pro Arg Lys Gln Thr
   145 150 155 160
   Asp Glu Ser Cys Thr Glu Lys Thr Val Glu Phe Met Glu Arg Met Gly
   165 170 175
   Lys Lys Pro Ile Val Val Lys Lys Asp Val Pro Gly Met Val Ala Asn
   180 185 190
   Arg Leu Ala Ala Ala Leu Trp Arg Glu Ala Val Asn Leu Val Tyr Gln
   195 200 205
   Gly Ile Ala Thr Pro Glu Glu Ile Asp Val Ala Val Lys Tyr Gly Pro
   210 215 220
   Gly Ile Arg Trp Ala Ile Thr Gly Val Tyr Leu Thr Tyr His Leu Gly
   225 230 235 240
   Gly Gly Gln Gly Gly Met Lys Tyr Phe Leu Glu Phe Phe Asp Asp His
   245 250 255
   Tyr Arg Arg Leu Trp Ser Asp Leu Ala Asn Trp Ser Glu Tyr Pro Glu
   260 265 270
   Gly Ser Phe Asp Ala Ile Leu Lys Ser Met Glu Gly Tyr Asp Ile Ile
   275 280 285
   Lys Arg Met Ser Tyr Asp Glu Leu Ala Lys Trp Arg Asp Asp Gln Leu
   290 295 300
   Ala Lys Ile Leu Lys Leu Ile Leu Glu Asp Gly
   305 310 315

## Beschreibung

Die vorliegende Erfindung betrifft Proteine, die eine enzymatische Aktivität zur Reduktion von substituierten Alkanonen, wie 3-Methylamino-1-(2-thienyl)-propan-1-on, aufweisen. Die Erfindung betrifft weiterhin Nukleinsäuren, die für diese Proteine kodieren, Nukleinsäurekonstrukte, Vektoren, genetisch veränderte Mikroorganismen sowie Verfahren zur Herstellung von substituierten (S)-Alkanolen, wie z.B. (S)-3-Methylamino-1-(2-thienyl)-(S)-propanol.

### Stand der Technik:

Dehydrogenasen sind vielseitig einsetzbare Katalysatoren zur enantioselektiven Reduktion von Aldehyden oder Ketonen zu den korrespondierenden Alkoholen. Man unterscheidet dabei (R)- und (S)-spezifische Dehydrogenasen. Diese Katalysatoren werden im verstärkten Maß für die industrielle Synthese von optisch aktiven Alkoholen eingesetzt. Optische Aktivität ist die Voraussetzung für die selektive Wirkung von vielen Pharma- und Agrowirkstoffen. Hier kann das eine Enantiomer die gewünschte, das andere Enantiomer eine genotoxische Wirkung haben. Aus diesem Grund werden in der Synthese von Pharma- und Agrowirkstoffen zur Herstellung von optisch aktiven Alkoholen Katalysatoren eingesetzt, die die notwendige Stereospezifität besitzen.

3-Methylamino-1-(2-thienyl)-(S)-propanol ("Duloxetinealkohol") ist Baustein in der Duloxetine-Synthese. Duloxetine ist ein Pharmawirkstoff, der sich momentan in der Zulassung befindet und im Indikationsgebiet Depression und Inkontinenz eingesetzt werden soll.

In der Literatur (vgl. EP-A-0 273 658) sind Synthesewege zum Duloxetinealkohol und Duloxetin beschrieben. Nachteil dieser Synthesewege ist, dass die Synthese zum racemischen Alkoholgemisch führt und anschließend eine Racematspaltung durch Oberführung des Racemats in eine Mischung aus Diastereomeren durch Salzbildung mit einem optisch aktiven Gegenion erforderlich ist. Anschließend erfolgte eine physikalischen Trennung der Diastereomere. Hieraus resultieren hohe Verfahrenskosten augfrund wiederholter Fest-Flüssigtrennungen und der erhöhte Einsatzstoffaufwand durch Zugabe eines optisch aktiven Salzes zur Trennung.

Einen preisgünstigeren Zugang zum Duloxetinealkohol würde die stereospezifische Reduktion des 3-Methylamino-1-(2-thienyl)-propanon bieten.

### Kurze Beschreibung der Erfindung:

Der Erfindung lag daher die Aufgabe zugrunde, eine Weg zur stereospezifischen Reduktion von substituierten Alkanonen, wie dem 3-Methylamino-1-(2-thienyl)-propan-2-on, zu finden.

Diese Aufgabe wurde durch den überraschenden Befund gelöst, dass Enzyme mit L-Carnitin Dehydrogenase-Aktivität zur stereospezifischen Katalyse der obigen Reaktion befähigt sind.

Ein erster Gegenstand der Erfindung betrifft ein Verfahren zur mikrobiologischen, insbesondere enantioselektiven Herstellung von substituierten (S)-Alkanolen der Formel I 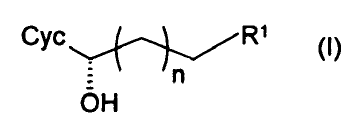 worin
- n: für einen ganzzahligen Wert von 0 bis 5, insbesondere 0, 1 oder 2, steht;
- Cyc: für einen gegebenenfalls substituierten einkernigen oder unsubstituierten mehrkernigen, gesättigten oder ungesättigten, carbocyclischen oder heterocyclischen Ring, insbesondere einen gegebenenfalls substituierten, ungesättigten, einkernigen heterocyclischen Ring, steht, und
- R¹: für Halogen, SH, OH, NO₂, NR²R³ oder NR²R³R⁴⁺X⁻, insbesondere Halogen oder NR²R³ steht, wobei R², R³ und R⁴ unabhängig voneinander für H oder einen Niedrigalkyl- oder Niedrigalkoxy-Rest stehen und X⁻ für ein Gegenion steht,
wobei man in einem ein Alkanon der Formel 11 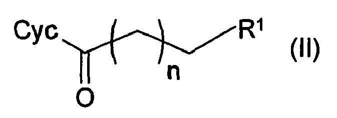 worin n, Cyc und R¹ die oben angegebenen Bedeutungen besitzen, enthaltenden Medium,
a) einen ein Enzym mit L-Carnitin Dehydrogenase-Aktivität produzierenden Mikroorganismus kultiviert, oder
b) ein Enzym mit L-Carnitin Dehydrogenase-Aktivität inkubiert,
wobei die Verbindung der Formel 11 zur Verbindung der Formel I enzymatisch reduziert wird, und man das in im Wesentlichen enantiomerenreiner Form gebildete Produkt isoliert.

In einer besonders bevorzugten Ausführungsform dient das Verfahren zur Herstellung von 3-Methylamino-1-(2-thienyl)-(S)-propanol der Formel III 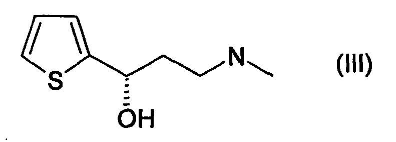 wobei man in einem 3-Methylamino-1-(2-thienyl)-propan-2-on der Formel IV 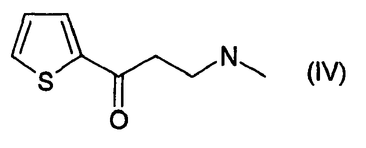 enthaltenden Medium diese Verbindung zur Verbindung der Formel III enzymatisch reduziert wird, und man das in im Wesentlichen enantiomerenreiner Form gebildete Produkt isoliert.

Vorzugsweise verwendet man bei diesen Verfahren ein Enzym mit L-Carnitin Dehydrogenase-Aktivität, das ausgewählt ist unter L-Carnitin Dehydrogenasen (E.C. 1.1.1.108) und 3-Hydroxyacyl-CoA Dehydrogenasen (E.C. 1.1.1.35).

Derartige Enzyme mit L-Carnitin Dehydrogenase-Aktivität sind insbesondere ausgewählt unter Enzymen aus Mikroorganismen der Gattungen Alcaligenes, Pseudomonas, Xanthomonas, Staphylococcus, Rhizobium, Agrobacterium, Streptomyces und Archaeglobus .

In einer besonders bevorzugten Ausführungsform des Verfahrens ist das Enzym mit L-Carnitin Dehydrogenase-Aktivität ausgewählt unter Enzymen, welche eine Aminosäuresequenz gemäß SEQ ID NO: 2, 3, 4, 5, 6, 7, 8, 9 oder 10 umfassen, oder von davon abgeleiteten Nukleinsäuresequenzen kodiert werden; und funktionalen Äquivalenten dieser Enzyme, welche L-Carnitin Dehydrogenase-Aktivität besitzen und die enantioselektive Synthese einer Verbindung der Formel katalysieren.

Beispielsweise kann das Enzym mit L-Carnitin Dehydrogenase-Aktivität von einem Nukleinsäuresequenz gemäß SEQ ID NO:1 oder einem funktionalen Äquivalent davon kodiert werden.

Vorzugsweise wird das erfindungsgemäße Verfahren unter Zugabe von Reduktionsäquivalenten (NADH oder NADPH) oder die bei der Umsetzung verbrauchten Reduktionsäquivalente regenerierenden (biochemischen oder elektrochemischen) Bedingungen durchgeführt.

Weiterhin ist es bevorzugt, die Umsetzung der Verbindung der allgemeinen Formel II, wie z.B. der Formel IV, in Gegenwart eines Mikroorganismus erfolgen zu lassen, der ausgewählt ist unter Bakterien der Familien Enterobacteriaceae, Pseudomonadaceae, Rhizobiaceae, Streptomycetaceae und Nocardiaceae. Der Mikroorganismus kann insbesondere ein rekombinanter Mikroorganismus sein, der mit einem Nukleinsäurekonstrukt transformiert ist, welche für ein Enzym mit L-Carnitin Dehydrogenase-Aktivität gemäß obiger Definition kodiert.

Gegenstand der Erfindung ist insbesondere ein Verfahren gemäß obiger Definition, wobei man
a) einen ein Enzym mit L-Carnitin Dehydrogenase-Aktivität produzierenden Mikroorganismus aus einer natürlichen Quelle isoliert oder rekombinant herstellt,
b) diesen Mikroorganismus vermehrt,
c) aus dem Mikroorganismus das Enzym mit L-Carnitin Dehydrogenase-Aktivität gegebenenfalls isoliert oder eine dieses Enzym enthaltende Proteinfraktion herstellt, und
d) den Mikroorganismus gemäß Stufe b) oder das Enzym gemäß Stufe c) in ein Medium überführt, das eine Verbindung der Formel I enthält.

Die Erfindung betrifft weiterhin ein Verfahren zur Herstellung einer Verbindung der allgemeinen Formel V 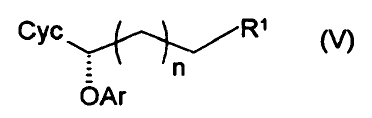 worin n, Cyc, und R¹ die oben angegebenen Bedeutungen besitzen und Ar für einen mehrkernigen oder gegebenenfalls substituierten einkernigen Aryl-Rest steht, und
wobei man
a) zunächst auf mikrobiologischem Weg gemäß der Definition in einem der vorhergehenden Ansprüche eine Verbindung der Formel I herstellt; und
b) die Verbindung der Formel I mit eine aromatischen Verbindung der Formel VI

   Ar-Y (VI)

   worin Ar die oben angegebenen Bedeutungen besitzt und Y für eine Abgangsgruppe steht, umsetzt und
c) die Verbindung der Formel V isoliert und gegebenenfalls in ein pharmazeutisch verträgliches Säureadditionssalz, wie z.B. Oxalate überführt.

Vorzugsweise stelle man dabei eine Verbindung der Formel V her, worin Ar für 1-Naphthyl, Cyc für 2-Thienyl, R¹ für Monomethylamino und n für 1 steht.

Ein weiterer Gegenstand der Erfindung betrifft Polypeptide, welche eine Aminosäuresequenz gemäß SEQ ID NO: 2, 3, 4, 5, 6, 7, 8, 9 oder 10 umfassen, oder von davon abgeleiteten Nukleinsäuresequenzen kodiert werden; und funktionale Äquivalente dieser Enzyme, welche L-Carnitin Dehydrogenase-Aktivität besitzen und die enantioselektive Synthese einer Verbindung der Formel I und/oder III katalysieren.

Gegenstand der Erfindung sind außerdem kodierende Nukleinsäuresequenzen, umfassend die kodierende Sequenz für ein Polypeptid gemäß obiger Definition.

Weiterhin betrifft die Erfindung Expressionskassetten, umfassend in operativer Verknüpfung mit wenigstens einer regulativen Nukleinsäuresequenz eine kodierende Nukleinsäuresequenz gemäß obiger Definition.

Ein weiterer Gegenstand der Erfindung sind rekombinante Vektoren, umfassend wenigstens eine solche Expressionskassette.

Die Erfindung betrifft auch prokaryotische oder eukaryotische Wirte, welche mit wenigstens einem erfindungsgemäßen Vektor transformiert sind.

Ein letzter Gegenstand der Erfindung betrifft die Verwendung eines Enzyms mit L-Carnitin Dehydrogenase-Aktivität gemäß obiger Definition oder eines dieses Enzym produzierenden Mikroorganismus zur Herstellung von Verbindungen der Formeln I oder III, wie insbesondere zur Herstellung von Duloxetine der Formel VII

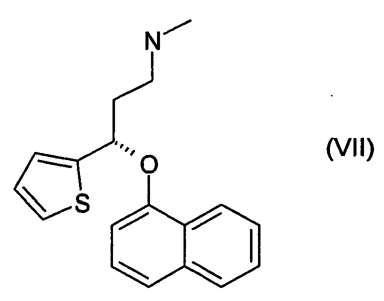

### Detaillierte Beschreibung der Erfindung:

### A. Allgemeine Begriffe und Definitionen

Werden keine andere Angaben gemacht, so gelten folgende allgemeine Bedeutungen:
"Halogen" steht für Fluor, Chlor, Brom oder Jod , insbesondere Fluor oder Chlor. "Niedrigalkyl" steht für geradkettige oder verzweigte Alkylreste 1 bis 6 C-Atomen, wie Methyl, Ethyl, i- oder n-Propyl, n-, i-, sec.- oder tert.-Butyl, n-Pentyl oder 2-MethylButyl, n-Hexyl, 2-Methyl-pentyl, 3-Methyl-pently, 2-Ethyl-butyl.
"Niedrigalkenyl" steht für die ein- oder mehrfach, vorzugsweise einfach oder zweifach ungesättigten Analoga oben genannter Alkylreste mit 2 bis 6 Kohlenstoffatomen, wobei die Doppelbindung in beliebiger Position der Kohlenstoffkette liegen kann.
"Niedrigalkoxy" steht für die Sauerstoff-terminierten Analoga obiger Alkylreste.
"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, ausgewählt unter Halogen, Niedrigalkyl, Niedrigalkoxy gemäß obiger Definition oder Trifluormethyl.

### B. Substituierte Alkanone, (S)-Alkanole und Derivate davon

Erfindungsgemäß durch enzymatische Katalyse zugängliche Alkanole sind solche obiger Formel (I) worin
- n: für einen ganzzahligen Wert von 0 bis 5 steht;
- Cyc: für einen gegebenenfalls substituierten, ein- oder mehrkernigen, gesättigten oder ungesättigten, carbocyclischen oder heterocyclischen Ring steht, und
- R¹: für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigalkoxy-Rest stehen und X⁻ für ein Gegenion steht.

Die zur enzymatischen Synthese verwendeten Alkanole obiger Formel II sind an sich bekannte Verbindungen und unter Anwendung allgemein bekannter organischer Syntheseverfahren zugänglich (vgl. z.B. EP-A- 0 273 658).

Vorzugsweise steht n in obigen Verbindungen für 0, 1 oder 2, insbesondere für 1.

Als Beispiele für carbo- und heterocyclische Gruppen Cyc sind insbesondere ein- oder zweikernigen, vorzugsweise einkernige, Gruppen mit bis zu 4, vorzugsweise 1 oder 2 gleichen oder verschiedenen Ring-Heteroatomen, ausgewählt unter O, N und S sind zu nennen:

Diese carbo- oder heterocyclischen Ringe umfassen insbesondere 3 bis 12, vorzugsweise 4, 5 oder 6 Ring-Kohlenstoffatomen. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; sowie 5- bis 7-gliedrige gesättigte oder ein oder mehrfach ungesättigte heterocyclische Reste mit 1 bis 4 Heteroatomen, die ausgewählt sind unter O, N und S, wobei der Heterocyclus gegebenenfalls mit einem weiteren Heterocyclus oder Carbocyclus kondensiert sein kann. Insbesondere sind zu nennen heterocyclische Reste, abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin.

Die Reste Cyc können dabei über eine beliebige Ringposition, vorzugsweise über ein Ring-Kohlenstoffatom, an das Alkanon bzw. das Alkanol gebunden sein.

Beispiele für geeignet Cyc-Reste sind 2-Thienyl, 3-Thienyl; 2-Furanyl, 3-Furanyl; 2-Pyridyl, 3-Pyridyl oder 4-Pyridyl; 2-Thiazolyl, 4-Thiazolyl oder 5-Thiazolyl; 4-Methyl-2-thienyl, 3-Ethyl-2-thienyl, 2-Methyl-3-thienyl, 4-Propyl-3-thienyl, 5-n-Butyl-2-thienyl, 4-Methyl-3-thienyl, 3-Methyl-2-thienyl; 3-Chlor-2-thienyl, 4-Brom-3-thienyl, 2-lod-3-thienyl, 5-lod-3-thienyl, 4-Fluor-2-thienyl, 2-Brom-3-thienyl, und 4-Chlor-2-thienyl.

Die Reste Cyc können weiterhin ein- oder mehrfach, wie z.B. ein- oder zweifach, substituiert sein. Vorzugsweise sitzen die Substituenten an einem Ring-Kohlenstoffatom. Beispiele für geeignete Substituenten sind Halogen, Niedrigalkyl, Niedrigalkenyl, Niedrigalkoxy, -OH, -SH, -NO₂ oder NR²R³, wobei R² und R³ obige Bedeutungen besitzen, bevorzugt Halogen oder Niedrigalkyl.

R¹ steht insbesondere für Halogen, NR²R³ oder NR²R³R⁴⁺X⁻, wobei R², R³ bzw. R², R³ und R⁴ unabhängig voneinander für H oder einen Niedrigalkyl- oder Niedrigalkoxy-Rest stehen und X⁻ für ein Gegenion steht, wobei vorzugsweise einer der Reste R², R³ und R⁴ für H steht. Geeignete Gegenionen sind beispielsweise Säureanionen, wie sie beispielsweise bei Herstellung eines Säureadditionssalzes anfallen. Beispiel hierzu sind z.B. in der EP-A-0 273 658 genannt, worauf hiermit Bezug genommen wird. Bevorzugte Beispiele für Reste R¹ sind insbesondere Fluor oder Chlor, sowie NR²R³ worin R² und R³ gleich oder verschieden sind und für H oder Methyl, Ethyl oder n-Propyl stehen; besonders bevorzugt steht R¹ für Chlor oder -NHMethyl.

### C. Enzyme mit L-Carnitin Dehydrogenase-Aktivität

Das erfindungsgemäße Enzym mit L-Carnitin Dehydrogenase-Aktivität ist insbesondere ausgewählt unter L-Carnitin Dehydrogenasen (E.C. 1.1.1.108) und 3-Hydroxyacyl-CoA Dehydrogenasen (E.C. 1.1.1.35) (vgl. auch Kleber HP (1997) FEMS Microbiology Letters 147 1- 9).

Diese L-Carnitin Dehydrogenasen / Hydroxyacyl-CoA Dehydrogenasen lassen sich in Organismen, insbesondere Mikroorganismen wie Bakterien, Hefen oder Pilzen, finden. Das Enzym bzw. die Enzyme besitzen eine hohe enzymatische Aktivität zur Reduktion von Alkanonen der Formel II, wie 3-Methylamino-1-(2-thienyl)-propan-1-on zu 3-Methylamino-1-(2-thienyl)-(S)-propanol. Andere Substrate, wie z.B. die Dimethylderivate des Ketons werden wie die Monomethylverbidungen ebenfalls von der Dehyrdogenase umgesetzt.

Ohne darauf beschränkt zu sein sind solche Enzyme bevorzugt aus Mikroorganismen der Gattungen Alcaligenes, Pseudomonas, Xanthomonas, Staphylococcus, Rhizobium, Agrobacterium, Streptomyces und Archaeglobus zugänglich.

Bevorzugte Enzym mit L-Carnitin Dehydrogenase-Aktivität umfassen eine Aminosäuresequenz gemäß SEQ ID NO: 2, 3, 4, 5, 6, 7, 8, 9 oder 10.

Erfindungsgemäß mit umfasst sind ebenfalls "funktionale Äquivalente" der konkret offenbarten Enzyme mit L-Carnitin Dehydrogenase-Aktivität und die Verwendung dieser in den erfindungsgemäßen Verfahren.

"Funktionale Äquivalente" oder Analoga der konkret offenbarten Ezyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die von 3-Methylamino-1-(2-thienyl)-propan-1-on zum entsprechenden S-Alkohol reduzieren und die mindestens 20 %, bevorzugt 50 %, besonders bevorzugt 75 %, ganz besonders bevorzugt 90 % der Aktivität eines Enzyms, umfassend eine der unter SEQ ID NO:2 bis 10 aufgeführten Aminosäuresequenz, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90%, 95% oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation oder Verkürzung des Proteins.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### D. Kodierende Nukleinsäuresequenzen

Die Begriffe "exprimieren" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Gegenstand der Erfindung sind insbesondere Nukleinsäuresequenzen, die für ein Enzym mit L-Carnitin Dehydrogenase-Aktivität kodieren. Bevorzugt sind Nukleinsäuresequenzen umfassend eine Sequenz gemäß SEQ ID NO:1; oder von den Aminosäuresequenzen gemäß SEQ ID NO.: 2 bis 10 abgeleitete Nukleinsäuresequenzen.

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die erfindungsgemäßen Nukleinsäuresequenzen lassen sich prinzipiell aus allen Organismen identifizieren und isolieren. Vorteilhaft lassen sich die erfindungsgemäßen Nukleinsäuresequenzen, wie SEQ ID NO: 1 oder die Homologen davon, aus Pilzen, Hefen oder Bakterien isolieren. Als Bakterien seien gram-negative und gram-positive Bakterien genannt. Bevorzugt werden die erfindungsgemäßen Nukleinsäuren aus gram-negativen Bakterien vorteilhaft aus alpha-Proteobacterien, beta-Proteobacterien oder gamma-Proteobacterien, besonders bevorzugt aus Bakterien der Familien Enterobacteriaceae, Pseudomonadaceae oder Rhizobiaceae, ganz besonders bevorzugt aus Bakterien der Gattung Agrobacterium, Pseudomonas oder Burkholderia über dem Fachmann bekannte Methoden isoliert.

Erfindungsgemäße Nukleinsäuresequenzen, wie SEQ ID No: 1 oder Derivate davon, Homologe oder Teile dieser Sequenzen, lassen sich beispielsweise mit üblichen Hybridisierungsverfahren oder der PCR-Technik aus anderen Pilzen oder Bakterien, z.B. über genomische oder cDNA-Banken, isolieren. Diese DNA-Sequenzen hybridisieren unter Standardbedingungen mit den erfindungsgemäßen Sequenzen. Zur Hybridisierung werden vorteilhaft kurze Oligonukleotide der konservierten Bereiche beispielsweise aus dem aktiven Zentrum, die über Vergleiche mit der L-Carnitin Dehydrogenase in dem Fachmann bekannter Weise ermittelt werden können, verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO: 1 sind beispielsweise Allelvarianten zu verstehen, die mindestens 40 % Homologie auf der abgeleiteten Aminosäureebene, bevorzugt mindestens 60 % Homologie, ganz besonders bevorzugt mindestens 80 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivate auch Homologe der erfindungsnemäßen Nukleinsäuresequenzen, insbesondere der SEQ ID NO: 1, beispielsweise pilzliche oder bakterielle Homologe, verkürzte Sequenzen, Einzelstrang-DNA oder RNA der kodierenden und nichtkodierenden DNA-Sequenz, zu verstehen. So besitzten z.B. Homologe zur der SEQ ID NO: 1 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 1 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Weiterhin umfasst die Erfindung auch Nukleinsäuresequenzen, welchen mit oben genannten kodierenden Sequenzen unter "stingenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können erfindungsgemäße Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1 % SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

### E. Erfindungsgemäße Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem erfindungsgemäßen Nukleinsäurekonstrukt sind insbesondere die L-Carnitin Dehydrogenasegene mit Sequenz SEQ ID NO: 1 und die Derivate und Homologen davon sowie die von SEQ ID NO: 2 bis 10 ableitbaren Nukleinsäuresequenzen zu verstehen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz (wie z.B. SEQ ID NO: 1 oder seine Homologen) insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, Ipp-, lac-, Ipp-lac-, Iacl^{q-} T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. In diesem Zusammenhang sind auch die Promotoren der Pyruvatdecarboxylase und der Methanoloxidase, beispielsweiseaus Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHIac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### F. Erfindungsgemäß brauchbare Wirte

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Erfindungsgemäß sind auch homolog rekombinierte Mikroorganismen herstellbar. Dazu wird ein Vektor hergestellt, der zumindest einen Abschnitt eines erfindungsgemäßen Gens oder einer kodierenden Sequenz enthält, worin gegebenenfalls wenigstens eine Aminosäure-Deletion, -Addition oder -Substitution eingebracht worden ist, um die erfindungsgemäße Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des erfindungsgemäßen Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit L-Carnitin Dehydrogenaseaktivität kodieren (Kleber HP (1997) FEMS Microbiology, 147, 1 - 9).

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

Die Wirtsorganismen enthalten vorteilhaft 1 U/I Enzymaktivität, wie z.B. L-Carnitinedehydrogenaseaktivität, bevorzugt 100 U/I, besonders bevorzugt mehr als 1000 U/I.

### G. Rekombinante Herstellung der Polypeptide:

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### H. Durchführung des erfindungsgemäßen Verfahrens zur Herstellung von (S)-Alkanolen

Die Enzyme mit L-Carnitin Dehydrogenase-Aktivität, wie Carnitin Dehydrogenase oder die Hydroxyacyl-CoA-Dehydrogenase, können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten, wie 3-Methylamino-1-(2-thienyl)-(S)-propanol, sind im erfindungsgemäßen Verfahren Enantiomere zu verstehen, die eine Enantiomerenanreicherung zeigen. Bevorzugt werden im Verfahren Enantiomerenreinheiten von mindestens 70 %ee, bevorzugt von min. 80 %ee, besonders bevorzugt von min. 90 %ee, ganz besonders bevorzugt min. 98 %ee erreicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder Enzyme verwendet, so werden diese vor der Extraktion zweckmäßigerweise abgetrennt beispielsweise über eine Filtration oder Zentrifugation.

Das im erfindungsgemäßen Verfahren hergestellte Produkt, wie 3-Methylamino-1-(2-thienyl)-(S)-propanol, läßt sich vorteilhaft aus der wässrigen Reaktionslösung über Extraktion oder Destillation oder vorteilhaft über Extraktion und Destillation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetyt, Diisopropylether, Benzol, MTBE oder Essigester, ohne darauf beschränkt zu sein.

Nach Einengen der organischen Phase können die Produkte in der Regel in guten chemischen Reinheiten, das heißt größer 80 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 °C bis 10 °C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende vorteilhafte mindestens einmalig durchgeführte Kristallisation kann die Enantiomerenreinheit des Produktes falls erforderlich weiter gesteigert werden.

Bei den genannten Aufarbeitungsarten lässt sich das Produkt des erfindungsgemäßen Verfahrens in Ausbeuten von 60 bis 100 %, bevorzugt von 80 bis 100 %, besonders bevorzugt von 90 bis 100 %, bezogen auf das für die Reaktion eingesetzte Substrat, wie z.B. von 3-Methylamino-1-(2-thienyl)-propan-1-on, isolieren. Das isolierte Produkt zeichnet sich durch eine hohe chemische Reinheit von > 90 %, bevorzugt > 95 % besonders bevorzugt von > 98 % aus. Weiterhin haben die Produkt eine hohe Enantiomerenreinheit, die vorteilhaft falls erforderlich durch die Kristallisation weiter gesteigert werden kann.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die obige Beschreibung und die nachstehenden Beispiele dienen nur der Verdeutlichung der Erfindung. Die für den Fachmann offensichtlichen, zahlreich möglichen Abwandlungen sind erfindungsgemäß ebenfalls umfasst.

### Experimenteller Teil:

### Beispiel 1: Klonierung der Carnitin- oder Hydroxyacyl-CoA-Dehydrogenasen via PCR-Amplifikation

Bakterien ausgewählt aus den Gattungen Alcaligenes, Pseudomonas, Xanthomonas, Agrobacterium, Mesorhizobium und Rhizobium, Streptomyces und Archaeglobus wurden 1 - 3 Tage in 25 ml Komplexmedium (z.B. HFP = 1 % Pepton, 1% Trypton, 0,5 % Hefeextrakt, 0,3 % NaCl) angezogen, geerntet, in Puffer gewaschen, resuspendiert (5 ml 50 mM Tris pH 7.0) und die genomische DNA mit Hilfe des QIAGEN-Genomic-tip-Systems der Fa. Qiagen präpariert. Anschließend erfolgte die PCR-Amplifikation der Carnitin- bzw. 3-Hydroxyacyl-CoA-Dehydrogenase-Gene. Hierzu wurden die dem Fachmann zugänglichen DNA-Sequenzen zu den Dehydrogenase-Sequenzen der Seq.ID 2-10 vom N- und C-Terminus (jeweils 25-30 bp) ausgewählt, wahlweise Restriktionsschnittstellen zur Klonierung angehängt und die entsprechenden Oligonukleotide synthetisiert. Die PCR-Reaktion wurde mit Pfu-Polymerase (Stratagene) oder Taq-Polymerase (Roche) durchgeführt. Für die PCR-Reaktionen wurde beispielsweise folgendes Temperatur-Programm durchgeführt:

95°C für 3 Minuten; 30 Zyklen mit 95°C für 45 sec., 55°C für 45 sec, und 72°C für 2 min. 50 sec.; 72°C für 10 min.; Verwahrung bei 4°C bis zum ersten Gebrauch. Alle PCR Produkte wurden durch Agarosegel-Electrophorese (E-Gel, Invitrogen) und Säulen- Chromatography (GFX-Kit, Pharmacia) gereinigt. Die Klonierung in Vektoren wie pBluescript KSII, pKK223-3 und pDHE19.2 erfolgte mit durch geeignete Restriktionsverdaus und Ligation nach Maniatis T, Fritsch EF and Sambrook J (1989) Molecular Cloning: A Laboratory Manual, Cold Spring Harbor Laboratory, Cold Spring Harbor (NY). In den pBADtopo-Vektor wurden Taq-PCR-Produkte direkt kloniert. Als geeignete Wirtsorganismen dienten E.coli XL1 B und TG1 (Stratagene).

### Beispiel 2: Klonierung der Carnitin- oder Hydroxyacyl-CoA-Dehydrogenasen durch Wachstumsselektion

Organismen der in Beispiel 1 genannten Gattungen und andere Bakterien, Hefen und Pilze sowie Isolate aus Bodenproben und E.coli-Genbanken, die durch Klonierung von DNA aus Bodenproben hergestellt wurden, wurden auf geeigneten Minimalmedien mit Carnitin oder Methylamino-1-(2-thienyl)-(S)-propanol, beispielweise 1% D,L-Carnitin, 0,2% K₂HPO₄ , 0,05% MgSO₄x 7 H₂O, 0,05% Hefe-Extrakt, ausgestrichen oder flüssig inkubiert, und nach einem Tag, drei Tagen, wöchentlich bzw. nach einem Monat in frisches Medium (wiederholt) überimpft. Die dadurch angereicherten Organismen wurden über Einzelkolonien oder Sortierung im Cell-Sorter isoliert. Sie zeigten die Fähigkeit, auf Carnitin oder Methylamino-1-(2-thienyl)-(S)-propanol als einziger C- und/oder N-Quelle zu wachsen. Die erhaltenen Stämmen konnten via PCR-Amplifikation (gemäß Beispiel 1) der Gewinnung neuer rekombinanter Dehydrogenase-Stämme dienen.

### Beispiel 3: Umsetzung von Methylamino-1-(2-thienyl)-(S)-propanol

Biomasse der unter Beispiel 1 und 2 erhaltenen Stämme wurde nach Anzucht in Gegenwart geeigneter Induktoren (z.B. 0,5 mM IPTG, 2 g/L Rhamnose, Carnitin) geerntet, in Puffer (z.B. 50 mM Tris-HCl pH 7.0) gewaschen und resuspendiert und die ruhenden Zellen mit NADH oder NADPH (0,1-5 mM), 1,6 mg - 50 mg Methylamino-1-(2-thienyl)-(S)-propanol und wahlweise Glucose oder Isopropanol (1-100 mol äq.) pro ml Ansatz versetzt und 1-24 h bei 30 °C inkubiert. Die Reaktion konnte durch Extinktionsabnahme bei 340 nm oder HPLC-Analytik verfolgt werden. Die Aktivitäten der Stämme lagen zwischen 0 und 100 U/I.

## Patentansprüche

1. Verfahren zur mikrobiologischen Herstellung von substituierten (S)-Alkanolen der Formel I 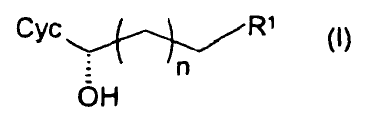 worin
n für einen ganzzahligen Wert von 0 bis 5 steht;
Cyc für einen gegebenenfalls substituierten, einkernigen oder unsubstituierten mehrkernigen, gesättigten oder ungesättigten, carbocyclischen oder heterocyclischen Ring steht, und
R¹ für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigalkoxy-Rest stehen und X⁻ für ein Gegenion steht,
wobei man in einem Alkanon der Formel II 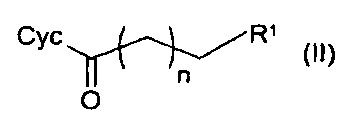 worin n, Cyc und R¹ die oben angegebenen Bedeutungen besitzen, enthaltenden Medium,
a) einen ein Enzym mit L-Carnitin Dehydrogenase-Aktivität produzierenden Mikroorganismus kultiviert, oder
b) ein Enzym mit L-Carnitin Dehydrogenase-Aktivität inkubiert,
wobei die Verbindung der Formel II zur Verbindung der Formel I enzymatisch reduziert wird, und man das gebildete Produkt isoliert.

2. Verfahren nach Anspruch 1, zur Herstellung einer Verbindung der Formel I, worin n und R¹ die oben angegebenen Bedeutungen besitzen und Cyc für einen gegebenenfalls substituierten, einkernigen, gesättigte oder ungesättigten, carbocyclischen oder heterocyclischen Ring steht.

3. Verfahren nach Anspruch 1, zur Herstellung von 3-Methylamino-1-(2-thienyl)-(S)-propanol der Formel III 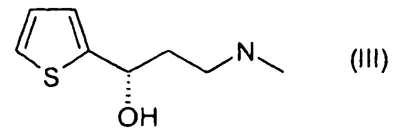 wobei man in einem 3-Methylamino-1-(2-thienyl)-propan-2-on der Formel IV 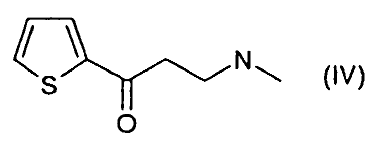 enthaltenden Medium diese Verbindung zur Verbindung der Formel III enzymatisch reduziert wird, und man das (in im Wesentlichen enantiomerenreiner Form) gebildete Produkt isoliert.

4. Verfahren nach einem der vorhergehenden Ansprüche, wobei das Enzym mit L-Carnitin Dehydrogenase-Aktivität ausgewählt ist unter L-Carnitin Dehydrogenasen (E.C. 1.1.1.108) und 3-Hydroxyacyl-CoA Dehydrogenasen (E.C. 1.1.1.35).

5. Verfahren nach einem der vorhergehenden Ansprüche, wobei das Enzym mit L-Carnitin Dehydrogenase-Aktivität ausgewählt ist unter Enzymen aus Mikroorganismen der Gattungen Alcaligenes, Pseudomonas, Xanthomonas, Staphylococcus, Rhizobium, Agrobacterium, Streptomyces und Archaeglobus.

6. Verfahren nach einem der vorhergehenden Ansprüche, wobei das Enzym mit L-Carnitin Dehydrogenase-Aktivität ausgewählt ist unter Enzymen, welche eine Aminosäuresequenz gemäß SEQ ID NO: 2, 3, 4, 5, 6, 7, 8, 9 oder 10 umfassen, oder von davon abgeleiteten Nukleinsäuresequenzen kodiert werden; und funktionalen Äquivalenten dieser Enzyme, welche L-Carnitin Dehydrogenase-Aktivität besitzen und die enantioselektive Synthese einer Verbindung der Formel I katalysieren.

7. Verfahren nach einem der vorhergehenden Ansprüche, wobei das Enzym mit L-Carnitin Dehydrogenase-Aktivität von einem Nukleinsäuresequenz gemäß SEQ ID NO:1 oder einem funktionalen Äquivalent davon kodiert wird.

8. Verfahren nach einem der vorhergehenden Ansprüche, wobei man die Umsetzung unter Zugabe von Reduktionsäquivalenten oder die bei der Umsetzung verbrauchten Reduktionsäquivalente regenerierenden Bedingungen durchführt.

9. Verfahren nach einem der vorhergehenden Ansprüche, wobei die Umsetzung der Verbindung der Formel II in Gegenwart eines Mikroorganismus erfolgt, der ausgewählt ist unter Bakterien der Familien Enterobacteriaceae, Pseudomonadaceae, Rhizobiaceae, Streptomycetaceae und Nocardiaceae.

10. Verfahren nach einem der vorhergehenden Ansprüche, wobei der Mikroorganismus ein rekombinanter Mikroorganismus ist, der mit einem Nukleinsäurekonstrukt transformiert ist, welche für ein Enzym mit L-Carnitin Dehydrogenase-Aktivität gemäß der Definition in einem der Ansprüche 4 bis 7 kodiert.

11. Verfahren nach einem der vorhergehenden Ansprüche, wobei man
a. einen ein Enzym mit L-Carnitin Dehydrogenase-Aktivität produzierenden Mikroorganismus aus einer natürlichen Quelle isoliert oder rekombinant herstellt,
b. diesen Mikroorganismus vermehrt,
c. aus dem Mikroorganismus das Enzym mit L-Carnitin Dehydrogenase-Aktivität gegebenenfalls isoliert oder eine dieses Enzym enthaltende Proteinfraktion herstellt, und
d. den Mikroorganismus gemäß Stufe b) oder das Enzym gemäß Stufe c) in ein Medium überführt, das eine Verbindung der Formel I enthält.

12. Verfahren zur Herstellung einer Verbindung der allgemeinen Formel V 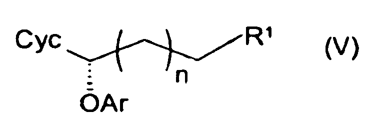 worin n, Cyc, und R¹ die oben angegebenen Bedeutungen besitzen und Ar für einen mehrkernigen oder gegebenenfalls substituierten einkernigen aromatischen Rest steht, und
wobei man
a) zunächst auf mikrobiologischem Weg gemäß der Definition in einem der vorhergehenden Ansprüche eine Verbindung der Formel I herstellt; und
b) die Verbindung der Formel I mit eine aromatischen Verbindung der Formel VI
Ar-Y (VI)
worin Ar die oben angegebenen Bedeutungen besitzt und Y für eine Abgangsgruppe steht, umsetzt und
c) die Verbindung der Formel V isoliert.

13. Verfahren nach Anspruch 12, wobei man eine Verbindung der Formel V herstellt, worin Ar für 1-Naphthyl, Cyc für 2-Thienyl, R¹ für Monomethylamino und n für 1 steht.

14. Verwendung eines Polypeptids, welches eine Aminosäuresequenz gemäß SEQ ID NO: 2, 3, 4, 5, 6, 7, 8, 9 oder 10 umfasst, oder von einer davon abgeleiteten Nukleinsäuresequenz kodiert wird; oder eines funktionale Äquivalents dieser Enzyme, welches L-Carnitin Dehydrogenase-Aktivität besitzt und die enantioselektive Synthese einer Verbindung der Formel I katalysieren, zur mikrobiellen oder enzymatischen Synthese von substituierten (S)-Alkanolen gemäß Formel I oder III.

15. Verwendung einer Nukleinsäuresequenz, umfassend die kodierende Sequenz für ein Polypeptid gemäß Anspruch 14; oder einer Expressionskassette, umfassend in operativer Verknüpfung mit wenigstens einer regulativen Nukleinsäuresequenz eine solche kodierende Nukleinsäuresequenz; oder eines rekombinanten Vektors, umfassend wenigstens eine solche Expressionskassette, zur Herstellung eines rekombinanten prokaryotischen oder eukaryotischen Wirts zur mikrobiellen Synthese von substituierten (S)-Alkanolen gemäß Formel I oder III.

16. Verwendung eines Enzyms mit L-Carnitin Dehydrogenase-Aktivität gemäß der Definition in einem der Ansprüche 4 bis 7 oder eines dieses Enzym produzierenden Mikroorganismus zur Herstellung von Verbindungen der Formeln I oder III.

17. Verwendung nach Anspruch 16 zur Herstellung von Duloxetine.

## Claims

1. A method for the microbiological preparation of substituted (S)-alkanols of the formula I 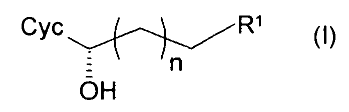 in which
n is an integer from 0 to 5;
Cyc is an optionally substituted, mononuclear or unsubstituted polynuclear, saturated or unsaturated, carbocyclic or heterocyclic ring, and
R¹ is halogen, SH, OH, NO₂, NR²R³ or NR²R³R⁴⁺X⁻, where R², R³ and R⁴ independently of one another are H or a lower alkyl or lower alkoxy radical and X⁻ is a counterion,
wherein, in a medium comprising an alkanone of the formula II 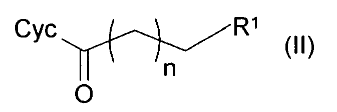 in which n, Cyc and R¹ are as defined above,
a) a microorganism producing an enzyme having L-carnitine dehydrogenase activity is cultured, or
b) an enzyme having L-carnitine dehydrogenase activity is incubated,
the compound of the formula II being enzymatically reduced to give the compound of the formula I, and the product formed is isolated.

2. The method according to claim 1 of preparing a compound of the formula I where n and R¹ are each as defined above and Cyc represents an optionally substituted, mononuclear, saturated or unsaturated, carbocyclic or heterocyclic ring.

3. The method according to claim 1 of preparing 3-methylamino-1-(2-thienyl)-(S)-propanol of the formula III 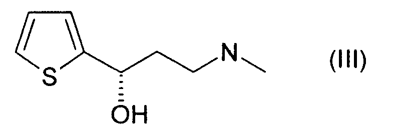 wherein, in a medium comprising 3-methylamino-1-(2-thienyl)-propan-2-one of the formula IV 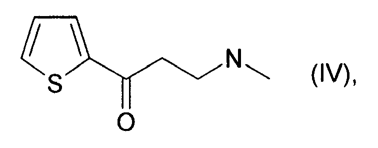 said compound is enzymatically reduced to give a compound of the formula III and the (essentially enantiomerically pure) product formed is isolated.

4. The method according to either of the preceding claims, wherein the enzyme having L-carnitine dehydrogenase activity is selected from among L-carnitine dehydrogenases (E.C. 1.1.1.108) and 3-hydroxyacyl-CoA dehydrogenases (E.C. 1.1.1.35).

5. The method according to any of the preceding claims, wherein the enzyme having L-carnitine dehydrogenase activity is selected from among enzymes of microorganisms of the genera Alcaligenes, Pseudomonas, Xanthomonas, Staphylococcus, Rhizobium, Agrobacterium, Streptomyces and Archaeglobus.

6. The method according to any of the preceding claims, wherein the enzyme having L-carnitine dehydrogenase activity is selected from among enzymes comprising an amino acid sequence according to SEQ ID NO: 2, 3, 4, 5, 6, 7, 8, 9 or 10 or encoded by nucleic acid sequences derived therefrom; and functional equivalents of said enzymes, which have L-carnitine dehydrogenase activity and catalyze the enantioselective synthesis of a compound of the formula I.

7. The method according to any of the preceding claims, wherein the enzyme having L-carnitine dehydrogenase activity is encoded by a nucleic acid sequence according to SEQ ID NO: or a functional equivalent thereof.

8. The method according to any of the preceding claims, wherein the reaction is carried out with addition of reduction equivalents or under conditions in which the reduction equivalents consumed are regenerated.

9. The method according to any of the preceding claims, wherein the compound of the formula II is reacted in the presence of a microorganism selected from among the bacteria of the families Enterobacteriaceae, Pseudomonadaceae, Rhizobiaceae, Streptomycetaceae and Nocardiaceae.

10. The method according to any of the preceding claims, wherein the microorganism is a recombinant microorganism which has been transformed with a nucleic acid construct coding for an enzyme having L-carnitine dehydrogenase activity as defined in any of claims 4 to 7.

11. The method according to any of the preceding claims, wherein
a. a microorganism producing an enzyme having L-carnitine dehydrogenase activity is isolated from a natural source or is prepared recombinantly,
b. said microorganism is propagated,
c. said enzyme having L-carnitine dehydrogenase activity is, if appropriate, isolated from said microorganism or a protein fraction comprising said enzyme is prepared from said microorganism, and
d. said microorganism according to stage b) or said enzyme according to stage c) is transferred into a medium comprising a compound of the formula I.

12. A method of preparing a compound of the formula V 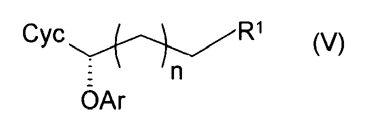 in which n, Cyc and R¹ are as defined above and Ar is a polynuclear or optionally substituted mononuclear aromatic radical, and
wherein
a) first a compound of the formula I is prepared microbiologically as defined in any of the preceding claims; and
b) the compound of the formula I is reacted with an aromatic compound of the formula VI
Ar-Y (VI)
in which Ar is as defined above and Y is a leaving group, and
c) the compound of the formula V is isolated.

13. The method according to claim 12, wherein a compound of the formula V in which Ar is 1-naphthyl, Cyc is 2-thienyl, R¹ is monomethylamino and n is 1 is prepared.

14. The use of a polypeptide which comprises an amino acid sequence according to SEQ ID NO: 2, 3, 4, 5, 6, 7, 8, 9 or 10 or is encoded by a nucleic acid sequence derived therefrom; or of a functional equivalent of these enzymes, which has L-carnitine dehydrogenase activity and catalyzes the enantioselective synthesis of a compound of the formula I, for microbial or enzymatic synthesis of substituted (S)-alkanols according to formula I or III.

15. The use of a nucleic acid sequence comprising the coding sequence for a polypeptide according to claim 14; or of an expression cassette comprising, in operative linkage with at least one regulatory nucleic acid sequence, such a coding nucleic acid sequence; or of a recombinant vector comprising at least one such expression cassette, for producing a recombinant prokaryotic or eukaryotic host for microbial synthesis of substituted (S)-alkanols according to the formula I or III.

16. The use of an enzyme having L-carnitine dehydrogenase activity as defined in any of claims 4 to 7 or of a microorganism producing said enzyme for preparing compounds of the formula I or III.

17. The use according to claim 16 for preparing duloxetine.

## Revendications

1. Procédé pour la fabrication microbiologique de (S)-alcanols substitués de formule I : 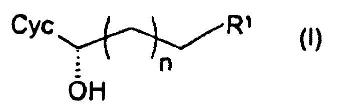 dans laquelle
n représente une valeur de nombre entier de 0 à 5 ;
Cyc représente un cycle carbocyclique ou hétérocyclique, saturé ou insaturé, monocyclique éventuellement substitué, ou polycyclique non substitué,
R¹ représente un halogène, un groupe SH, OH, NO₂, NR²R³ ou NR²R³R⁴⁺X⁻, les radicaux R², R³ et R⁴ représentant, indépendamment les uns des autres, l'hydrogène ou un radical alkyle inférieur ou alcoxy inférieur et X⁻ représentant un contre-ion,
dans lequel on effectue, dans un milieu contenant une alcanone de formule II : 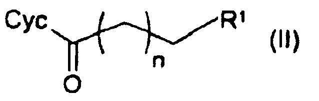 où n, Cyc et R¹ ont les significations indiquées ci-dessus,
a) une culture d'un micro-organisme producteur d'une enzyme exerçant une activité de L-carnitine déshydrogénase, ou
b) une incubation avec une enzyme exerçant une activité de L-carnitine déshydrogénase,
le composé de formule II étant réduit en composé de formule I par voie enzymatique, et on isole le produit formé.

2. Procédé selon la revendication 1, pour la fabrication d'un composé de formule I, dans laquelle n et R¹ ont les significations indiquées ci-dessus et le terme Cyc représente un cycle carbocyclique ou hétérocyclique, saturé ou insaturé, monocyclique, éventuellement substitué.

3. Procédé selon la revendication 1, pour la fabrication de 3-méthylamino-1-(2-thiényl)-(S)-propanol de formule III : 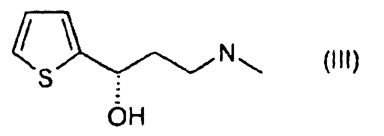 dans lequel on effectue, dans un milieu contenant une 3-méthylamino-1-(2-thiényl)-propan-2-one de formule IV : 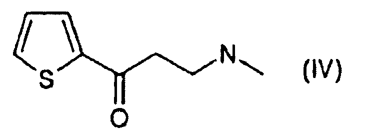 la réduction par voie enzymatique de ce composé en composé de formule III, et on isole le produit formé (sous une forme sensiblement pure au plan énantiomère).

4. Procédé selon l'une quelconque des revendications précédentes, dans lequel l'enzyme exerçant une activité de L-carnitine déshydrogénase est choisie parmi les L-carnitine déshydrogénases (E.C. 1.1.1.108) et les 3-hydroxyacyl-CoA déshydrogénases (E.C. 1.1.1.35).

5. Procédé selon l'une quelconque des revendications précédentes, dans lequel l'enzyme exerçant une activité de L-carnitine déshydrogénase est choisie parmi des enzymes provenant de micro-organismes des souches Alcaligenes, Pseudomonas, Xanthomonas, Staphylococcus, Rhizobium, Agrobacterium, Streptomyces et Archæglobus.

6. Procédé selon l'une quelconque des revendications précédentes, dans lequel l'enzyme exerçant une activité de L-carnitine déshydrogénase est choisie parmi des enzymes comprenant une séquence d'acides aminés représentée par les SEQ ID n° 2, 3, 4, 5, 6, 7, 8, 9 ou 10, ou qui sont codées par des séquences d'acide nucléique dérivées de celle-ci ; et parmi des équivalents fonctionnels de ces enzymes, qui exercent une activité de L-carnitine déshydrogénase et catalysent la synthèse énantiosélective d'un composé de formule I.

7. Procédé selon l'une quelconque des revendications précédentes, dans lequel l'enzyme exerçant une activité de L-carnitine déshydrogénase est codée par une séquence d'acide nucléique selon la SEQ ID n° 1 ou un équivalent fonctionnel de celle-ci.

8. Procédé selon l'une quelconque des revendications précédentes, dans lequel on réalise la réaction en ajoutant des équivalents de réduction ou en se plaçant dans les conditions qui régénèrent les équivalents de réduction consommés au cours de la réaction.

9. Procédé selon l'une quelconque des revendications précédentes, dans lequel on effectue la réaction du composé de formule II en présence d'un micro-organisme, qui est choisi parmi des bactéries des familles Enterobacteriaceæ, Pseudomonadaceæ, Rhizobiaceæ, Streptomycetaceæ et Nocardiaceæ.

10. Procédé selon l'une quelconque des revendications précédentes, dans lequel le micro-organisme est un micro-organisme recombinant qui a été transformé avec une construction d'acide nucléique, qui code pour une enzyme exerçant une activité de L-carnitine déshydrogénase selon la définition dans l'une quelconque des revendications 4 à 7.

11. Procédé selon l'une quelconque des revendications précédentes, dans lequel :
a. on isole un micro-organisme producteur d'une enzyme exerçant une activité de L-carnitine désydrogénase à partir d'une source naturelle ou on produit un recombinant,
b. on amplifie ce micro-organisme,
c. on isole éventuellement du micro-organisme l'enzyme exerçant une activité de L-carnitine déshydrogénase ou on produit une fraction protéique contenant cette enzyme, et
d. on transfère le micro-organisme de l'étape b) ou l'enzyme de l'étape c) dans un milieu, qui contient un composé de formule I.

12. Procédé pour fabriquer un composé de formule générale V : 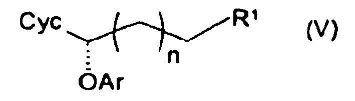 où n, Cyc, et R¹ ont les significations indiquées ci-dessus et Ar représente un radical aromatique polycyclique ou monocyclique, éventuellement substitué, et
dans lequel
a) on fabrique d'abord, par voie microbiologique et selon la définition dans l'une quelconque des revendications précédentes, un composé de formule I ; et
b) on fait réagir le composé de formule I avec un composé aromatique de formule VI :
Ar-Y (VI)
dans laquelle Ar a la signification indiquée ci-dessus et Y représente un groupe partant, et
c) on isole le composé de formule V.

13. Procédé selon la revendication 12, dans lequel on fabrique un composé de formule V, où Ar représente un groupe 1-naphtyle, Cyc représente un groupe 2-thiényle, R¹ représente un groupe monométhylamino et n est égal à 1.

14. Utilisation d'un polypeptide, qui comprend une séquence d'acides aminés représentée par les SEQ ID n° 2, 3, 4, 5, 6, 7, 8, 9 ou 10, ou qui est codé par une séquence d'acide nucléique dérivée de celle-ci ; ou d'un équivalent fonctionnel de ces enzymes qui exerce une activité de L-carnitine déshydrogénase et catalyse la synthèse énantiosélective d'un composé de formule I, pour la synthèse microbienne ou enzymatique de (S)-alcanols substitués selon la formule I ou III.

15. Utilisation d'une séquence d'acide nucléique, comprenant la séquence codante pour un polypeptide selon la revendication 14 ; ou d'une cassette d'expression comprenant, en étant reliée de manière opérationnelle avec au moins une séquence d'acide nucléique régulatrice, une telle séquence d'acide nucléique codante ; ou d'un vecteur recombinant comprenant au moins une telle cassette d'expression, pour fabriquer un hôte procaryote ou eucaryote recombinant pour la synthèse microbienne de (S)-alcanols substitués selon la formule I ou III.

16. Utilisation d'une enzyme exerçant une activité de L-carnitine déshydrogénase selon la définition dans l'une quelconque des revendications 4 à 7 ou d'un micro-organisme producteur de cette enzyme pour fabriquer des composés de formule I ou III.

17. Utilisation selon la revendication 16 pour la fabrication de duloxétine.
